# EUROPEAN PATENT SPECIFICATION

(11) **EP 4 567 774 B1**
(45) Date of publication and mention of the grant of the patent: **19.08.2026**
(21) Application number: 24216276.6
(22) Date of filing: 28.11.2024
(51) Int. Cl.: G09B 23/30

(54) **MODEL EYE HAVING POSTERIOR CORNEA REFLECTIVITY SIMILAR TO HUMAN EYE**
AUGENMODELL MIT POSTERIORER HORNHAUTREFLEKTIVITÄT ÄHNLICH DEM MENSCHLICHEN AUGE
MODÈLE D'OEIL AYANT UNE RÉFLECTIVITÉ DE CORNÉE POSTÉRIEURE SEMBLABLE À L'OEIL HUMAIN

(30) Priority: 04.12.2023 KR 20230173411
(43) Date of publication of application: 11.06.2025
(73) Proprietor: Huvitz Co., Ltd., Anyang-si, Gyeonggi-do 14055 (KR)
(72) Inventor: JU, Ye Na, 14055 Anyang-si (KR); JO, Sunhyung, 14055 Anyang-si (KR); BAEK, Woo Kyung, 14055 Anyang-si (KR)
(74) Representative: IPAZ

(56) References cited:
- JP-A- 2000 237 142
- KR-A- 20100 121 927
- KR-A- 20120 115 807
- KR-A- 20220 152 655
- US-A1- 2019 213 917

## Description

### CROSS-REFERENCE TO RELATED APPLICATION

This application claims benefit of priority to Korean Patent Application No. 10-2023-0173411 filed on December 04, 2023.

### FIELD OF THE DISCLOSURE

The present disclosure relates to a model eye and, more particularly, to a model eye having a posterior cornea reflectivity similar to that of a human eye.

### BACKGROUND

US2019/213917A1 discloses an eye model according to the prior art.

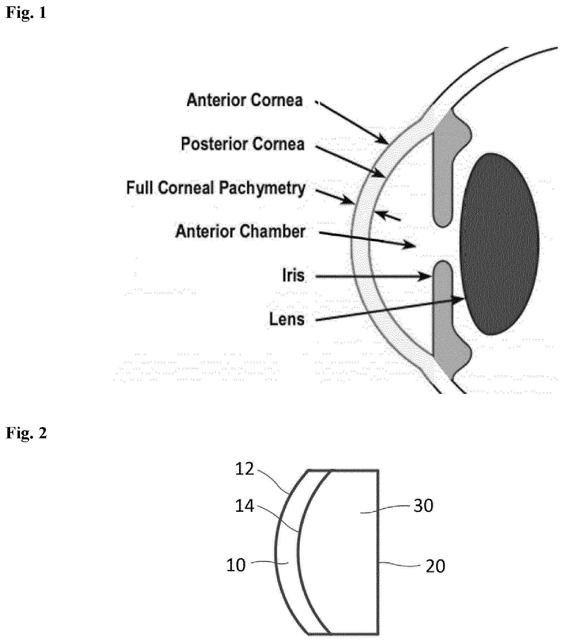

FIG. 1 is a diagram to explain the structure of the anterior part of a typical human eye. As shown in FIG. 1, the anterior part of the human eye is composed of the cornea located in the outermost layer, the iris located in the middle layer of the eye to control the amount of light, and the lens corresponding to the lens of a camera. The anterior chamber, the space between the cornea and the lens, is filled with a clear, water-like liquid.

The average curvature of the anterior cornea is approximately 7.8 mm, and the average curvature of the posterior cornea is approximately 6.5 mm.

Meanwhile, corneal thickness measurement using an optometry instrument (full cornea pachimetry) is performed by calculating the width using signals from the front and back of the cornea that are reflected after projecting a slit beam toward the cornea. In this process, when light passes through different media, the reflection coefficient (ρ) of the light changes depending on the refractive indexes of the media. When light is incident through medium 1 and reflected vertically from medium 2, the reflection coefficient (ρ) of light at the boundary between medium 1 and medium 2 may be calculated according to Equation 1 below.$ρ = \frac{{n}_{1} - {n}_{2}}{{n}_{1} + {n}_{2}}$

In Equation 1 above, n₁ is the refractive index of the region where light is incident (medium 1), and n₂ is the refractive index of the region where light is reflected (medium 2). The medium in front of the cornea is air with a refractive index (n) of approximately 1, the refractive index of the cornea is 1.3771, and the medium behind the cornea is a liquid with a refractive index of approximately 1.3374.

In the related art, to develop equipment to examine various symptoms or diseases of the cornea, model eyes that can refer to the shape of the cornea have been used, but there is no model eye that has the reflectivity of the anterior and posterior cornea similar to that of a human eye. That is, in the related art, model eyes have been utilized in which glass with a curvature similar to that of the cornea is used as a substitute for the cornea and the back of the cornea i.e. the anterior chamber is filled with water.

Table 1 below shows the refractive indices and reflection coefficients of the anterior cornea and posterior cornea of a human eye and the refractive indices and reflection coefficients of a related-art model eye.

**[Table 1]**

| | | | Air | Anterior cornea | Posterior Cornea |
|---|---|---|---|---|---|
| Human eye | | Refractive index (n) | 1 | 1.3771 | 1.3374 |
| | | Reflection coefficient | | **0.159** | **0.015** |
| Glass No. 1 (Fused silica) n= 1.458 | Water | Refractive index (n) | 1 | 1.458 | 1.3332 |
| | | Reflection coefficient | | 0.186 | **0.045** |

As can be seen in Table 1, for the human eye, the refractive index and the reflection coefficient at the anterior cornea are approximately 1.3771 and 0.159, and the refractive index and the reflection coefficient at the posterior cornea are approximately 1.3374 and 0.015. On the other hand, for the related-art model eye, the refractive index of the quartz glass (fused silica) and the reflection coefficient at the front of the quartz glass are approximately 1.458 and 0.186, and the refractive index of the water behind the quartz glass and the reflection coefficient at the back of the quartz glass are 1.3332 and 0.045. There are large deviations from the reflection coefficients of the anterior cornea and posterior cornea, so there is a problem in that good corneal signals cannot be obtained from the related-art model eye.

### [Prior Art Documents]

(Patent document 1) Korean Patent Publication No. KR 10-2012-0115807 A
(Patent document 2) Korean Patent Publication No. KR 10-2010-0121927 A
(Patent document 3) Korean Patent Publication No. KR 10-2022-0152655 A

### SUMMARY OF THE DISCLOSURE

### TECHNICAL OBJECTS

An object of the present disclosure is to provide a model eye having a posterior cornea reflectivity similar to that of a human eye.

### TECHNICAL SOLUTION

To achieve the above objectives, the model eye of the present disclosure having a posterior cornea reflectivity similar to that of a human eye may include: a body having a space formed therein; solid glass mounted on the front of the body to seal the body, as a substitute for the cornea of a human eye; and a liquid substance being vegetable oil filled in the space of the body.

The liquid substance may be sunflower oil.

The glass may be H-FK95 glass.

### EFFECTS OF THE DISCLOSURE

According to the model eye of the present disclosure, the reflection coefficients of the front and back surfaces of the glass of the model eye are similar to the reflection coefficients of the anterior cornea and the posterior cornea of a human eye, so that a higher quality measurement signal can be obtained when developing ophthalmic equipment such as a corneal thickness measurement instrument, and it can also be usefully utilized for the calibration of an optometric instrument.

### BRIEF DESCRIPTION OF THE DRAWINGS

FIG. 1 is a diagram describing the anterior structure of a human eye.
FIG. 2 is a schematic cross-sectional view of a model eye having a posterior cornea reflectivity similar to that of a human eye according to the present disclosure.

### DETAILED DESCRIPTION OF THE PREFERRED EMBODIMENTS

Hereinafter, preferred embodiments of the present disclosure will be described in detail with reference to the accompanying drawings.

FIG. 2 is a schematic cross-sectional view of a model eye having a posterior cornea reflectivity similar to that of a human eye according to the present disclosure.

As shown in FIG. 2, the model eye of the present disclosure is composed of a body 20 filled with a liquid substance 30, preferably vegetable oil, in a space corresponding to the anterior chamber of a human eye, and solid glass 10 corresponding to the cornea of the human eye and mounted on the front surface of the body 20.

In the above-described configuration, the body 20 may be implemented with a metal material or a plastic material. The glass 10 seals the body 20 to prevent the liquid substance 30 filled in the body 20 from leaking, and its front surface 12 and back surface 14 are formed to have curvatures corresponding to the anterior and posterior corneal curvatures of a human eye.

Table 2 below shows the refractive index of vegetable oils that may be filled in the internal space of the body 20.

**[Table 2]**

| | |
|---|---|
| Coconut oil | 1.452 |
| Olive oil | 1.466 |
| Canola oil | 1.474 |
| Sunflower oil | 1.475 |

As shown in Table 2, it may be seen that the refractive indices of representative vegetable oils are in the range of approximately 1.45 to 1.48.

Table 3 below is a table comparing the refractive index and reflection coefficient of the model eye of the present disclosure, when various types of glass and sunflower oil filling materials are adopted, with the reflection coefficient of the cornea of a human eye.

**[Table 3]**

| | | | Air | Anterior cornea | Posterior cornea |
|---|---|---|---|---|---|
| Human eye | | Refractive index (n) | 1 | 1.3771 | 1.3374 |
| | | Reflection coefficient | | 0.159 | 0.015 |
| Glass No. 1 (Fused silica) n= 1.458 | Water | Refractive index (n) | 1 | 1.458 | 1.3332 |
| | | Reflection coefficient | | 0.186 | 0.045 |
| | Sunflower oil | Refractive index (n) | 1 | 1.458 | 1.475 |
| | | Reflection coefficient | | 0.186 | 0.006 |
| Glass No. 2 (H-FK95) n= 1.438 | Water | Refractive index (n) | 1 | 1.438 | 1.3332 |
| | | Reflection coefficient | | 0.180 | 0.038 |
| | Sunflower oil | Refractive index (n) | 1 | 1.438 | 1.475 |
| | | Reflection coefficient | | 0.180 | 0.013 |
| Glass No. 3 (H-FK71) n= 1.457 | Water | Refractive index (n) | 1 | 1.457 | 1.3332 |
| | | Reflection coefficient | | 0.186 | 0.044 |
| | Sunflower oil | Refractive index (n) | 1 | 1.457 | 1.475 |
| | | Reflection coefficient | | 0.186 | 0.006 |

As shown in Table 3, the refractive index of fused silica is 1.458, the refractive index of H-FK95 glass is 1.438, and the refractive index of H-FK71 glass is 1.457, so it can be seen that the refractive index of the glass that can be used in the present disclosure is in the range of approximately 1.4 to 1.5, specifically, 1.43 to 1.46.

Meanwhile, comprehensively considering the reflection coefficients of the anterior cornea and the posterior cornea of a human eye, it can be seen that when glass 10 having a refractive index of 1.43 to 1.45, for example, H-FK95 glass 10 having a refractive index of 1.438, is adopted as a corneal substitute, and a liquid substance 30 having a refractive index of 1.465 to 1.485, for example, sunflower oil having a refractive index of 1.475, is adopted as a filling material 30 of the anterior chamber space, the deviation between the reflection coefficients of the front and back surfaces of the glass of the model eye and the reflection coefficients of the anterior cornea and the posterior cornea of the human eye is minimized.

According to the model eye of the present disclosure, the reflection coefficients of the front and back surfaces of the glass of the model eye are similar to the reflection coefficients of the anterior cornea and the posterior cornea of a human eye, so that a higher quality measurement signal can be obtained when developing ophthalmic equipment such as a corneal thickness measurement instrument, and it can also be usefully utilized for the calibration of an optometric instrument.

Although the embodiments of the present disclosure have been described above, the present disclosure is not limited to those embodiments described above.

The scope of the invention is defined in the appended claims.

### [DESCRIPTION OF REFERENCE SYMBOLS]

| | | | |
|---|---|---|---|
| 10: | corneal replacement glass | 12: | front surface of glass |
| 12: | front surface of glass | 20: | body |
| 30: | liquid filling substance | | |

## Claims

1. A model eye having a posterior cornea reflectivity similar to that of a human eye, comprising:
a body (20) having a space formed therein, said space corresponding to an anterior chamber of the human eye;
solid glass (10) mounted in front of the body (20) to seal the body (20), as a substitute for a cornea of a human eye; and
a liquid substance (30) being vegetable oil filled in the space of the body (20);
**characterized in that** a refractive index of the liquid substance (30) is between 1.465 and 1.485, and a refractive index of the glass (10) is between 1.43 and 1.45.

2. The model eye of claim 1, wherein the liquid substance (30) is sunflower oil.

3. The model eye of claim 1, wherein the glass (10) is H-FK95 glass.

## Patentansprüche

1. Modellauge, das ein Reflexionsvermögen einer hinteren Hornhaut ähnlich dem eines menschlichen Auges aufweist, umfassend:
einen Körper (20), der einen darin ausgebildeten Raum aufweist, wobei der Raum einer vorderen Kammer des menschlichen Auges entspricht;
massives Glas (10), das vor dem Körper (20) angebracht ist, um den Körper (20) abzudichten, als einen Ersatz für eine Hornhaut eines menschlichen Auges; und
eine flüssige Substanz (30), bei der es sich um Pflanzenöl handelt, das in den Raum des Körpers (20) eingefüllt ist;
**dadurch gekennzeichnet, dass** ein Brechungsindex der flüssigen Substanz (30) zwischen 1,465 und 1,485 liegt und ein Brechungsindex des Glases (10) zwischen 1,43 und 1,45 liegt.

2. Modellauge nach Anspruch 1, wobei es sich bei der flüssigen Substanz (30) um Sonnenblumenöl handelt.

3. Modellauge nach Anspruch 1, wobei es sich bei dem Glas (10) um H-FK95-Glas handelt.

## Revendications

1. Œil modèle ayant une réflectivité de cornée postérieure similaire à celle d'un œil humain, comprenant :
un corps (20) ayant un espace formé à l'intérieur, ledit espace correspondant à une chambre antérieure de l'œil humain ;
une plaque de verre solide (10) montée devant le corps (20) pour sceller le corps (20), en tant que substitut à une cornée d'un œil humain ; et
une substance liquide (30) étant de l'huile végétale remplissant l'espace du corps (20) ;
**caractérisé en ce qu'**un indice de réfraction de la substance liquide (30) est entre 1,465 et 1,485, et un indice de réfraction du verre (10) est entre 1,43 et 1,45.

2. Œil modèle selon la revendication 1, dans lequel la substance liquide (30) est de l'huile de tournesol.

3. Œil modèle selon la revendication 1, dans lequel le verre (10) est du verre H-FK95.
